# EUROPÄISCHE PATENTSCHRIFT

(11) **EP 0 374 725 B1**
(45) Veröffentlichungstag und Bekanntmachung des Hinweises auf die Patenterteilung: **18.08.1993**
(21) Anmeldenummer: 89123091.4
(22) Anmeldetag: 14.12.1989
(51) Int. Cl.: A61L 15/16, A61K 9/70, A61M 37/00

(54) **Transdermales therapeutisches System mit Norpseudoephedrin als aktivem Bestandteil**
Transdermal therapeutic system having norpseudoephedrine as the active compound
Système thérapeutique transdermique pour la norpseudoephédrine

(30) Priorität: 22.12.1988 DE 3843237
(43) Veröffentlichungstag der Anmeldung: 27.06.1990
(73) Patentinhaber: LTS Lohmann Therapie-Systeme GmbH & Co. KG, 56567 Neuwied (DE)
(72) Erfinder: Hille, Thomas, Dr., D-5450 Neuwied 1 (DE)
(74) Vertreter: Flaccus, Rolf-Dieter, Dr.

(56) Entgegenhaltungen:
- EP-A- 0 156 080
- EP-A- 0 195 643
- EP-A- 0 261 402
- EP-A- 0 305 756
- FR-A- 2 497 457

## Beschreibung

Die Erfindung betrifft ein transdermales therapeutisches System, das als aktiven Bestandteil Norpseudoephedrin enthält.

Die Amphetamine, die chemische Gruppe, in die viele Antiadiposita gehören, wurden schon in den 30er Jahren entdeckt, zunächst aber als Mittel gegen Müdigkeit verordnet. Erst später erkannte man, daß diese Arzneistoffe noch einen anderen therapeutischen nutzbaren Effekt haben - sie reduzieren das Hungergefühl. Durch gezielte Arzneistoffsynthese gelang es, Substanzen zu entwickeln, bei denen, die Reduktion des Hungergefühls in den Vordergrund tritt, während die stimulierende Komponente in den Hintergrund verlagert wird. Dennoch müssen bei Applikationen derartiger Antiadiposita folgende Gesichtspunkte berücksichtigt werden:
- Sie dürfen nicht mit bestimmten anderen Medikamenten (und nicht mit Alkohol) kombiniert werden.
- Die Gewohnheitsbildung und Suchtgefahr sind zu beachten.
- Intravenöse Injektionen sind wegen der Gefahr der Induktion von Blutspiegelspitzen zu vermeiden.

Gesetzgeber und pharmazeutische Industrie tragen dem gleichermaßen Rechnung, indem zum einen flüssige Arzneiformen der Rezeptpflicht unterliegen, während die festen derselben dem OTC-Sektor angehören, zum anderen aber auch durch die Entwicklung von oralen Depotformen die Dosierung von Antiadiposita gesenkt, deren Blutspiegel aber konstant gehalten werden kann. Da unter Medizinern in jüngster Zeit die These vertreten wird, daß zum Suchtpotential eines Wirkstoffes die Arzneiform beiträgt (ein konstanter Blutspiegel ist erheblich günstiger als der rasche Wechsel von Blutspiegelspitzen mit Überdosis zu Zeitintervallen mit Unterversorgung), liegt es nahe, solche Retardformen zu entwickeln, die noch besser als orale Depotformen ein konstantes Konzentrationsplateau erzeugen. Denn obwohl die gängigen oralen Depotformen galenisch-technologische Meisterleistungen sind, die unter in vitro-Bedingungen, d.h. in künstlichem Magen- bzw. Darmsaft optimale Freisetzungsprofile erzeugen, können sie dennoch keine konstanten Blutspiegel garantieren, da die Pharmakonresorption aus dem Gastrointestinaltrakt entscheidend beeinflußt wird durch die Magenentleerungszeit, die bei der Nahrungsaufnahme in Abhängigkeit vom Volumen und von der Nahrungszusammensetzung verzögert wird. Außerdem kommt es bei Nahrungsaufnahme zu pH-Wert-Verschiebungen im Magen und durch die Vermischung des Magensaftes mit dem Speisebrei zu eimer Abnahme diffusionswirksamer Gradienten. Die Bedeutung der pH-Wert-Bedingungen im Magen für die gastrale Resorption wird auch durch Untersuchungen, bei denen der Magensaft alkalisiert wurde, unterstrichen. So wurde in Tierversuchen nachgewiesen, daß nach Alkalisieren des Mageninhaltes mit NaHCO₃ auf pH : 8,0, die Resorption aus dem Magen bei schwachen Säuren entsprechend ihrem pKa-Wert reduziert und bei schwachen Basen erhöht wird. Da Amphetamine mittelstarke Basen sind (pkₐ-Wert D-Norpseudoephedrin : 8,9), ist der Effekt der pH-wertabhängigen Resorption zwar nicht stark ausgeprägt, keineswegs aber vernachlässigbar.

Zur Erzeugung einer möglichst konstanten Wirkstoffkonzentration ist der Einsatz sogenannter therapeutischer Systeme geeignet, die definiert sind als eine arzneistoffenthaltende Vorrichtung bzw. eine Darreichungsform, welche einen Arzneistoff oder mehrere in vorausbestimmter Rate kontinuierlich über einen festgelegten Zeitraum an einen festgelegten Anwendungsort abgibt (siehe Heilmann, "Therapeutische Systeme", 4.Aufl.,Enke Verlag Stuttgart 1984, Seite 26). Ein orales therapeutisches System, wie beispielsweise im EP 0 237 159 beschrieben, ist aber keine Lösung des geschilderten Problems, da sich mittelstarke Basen im sauren Magensaft besser lösen als im neutralen. Dadurch wird die Freisetzung pH-wert-abhängig.

In der US-PS 4,292,301 ist schon die transdermale Verabreichung von Ephedrin aus einer nichtklebenden Polymermatrix beschrieben, doch kommt dem Ephedrin nur eine schwache Wirkung als Antiadipositum zu.

Aufgabe der Erfindung ist es daher, Norpseudoephedrin als Antiadipositum bzw. ein Mittel in einer Form bereitzustellen, in der gegenüber der oralen Depotform ein weit verbesserter, konstanter Wirkstoffspiegel erzielt wird, um damit die Nachteile des Standes der Technik zu überwinden.

Diese Aufgabe wird erfindungsgemäß in überraschender Weise dadurch gelöst, daß ein transdermales therapeutisches System zur Verabreichung eines Antiadipositums an die Haut aus einer wirkstoffundurchlässigen Rückschicht, einer Reservoirschicht und gegebenenfalls einer wiederablösbaren Schutzschicht bereitgestellt wird, dadurch gekennzeichnet, daß die Reservoirschicht haftklebend ist und 10 - 90 Gew.-% Polymermaterial, 0-30 Gew-% Weichmacher und 0,1 - 20 Gew.-% Norpseudoephedrin enthält.

Diese Lösung ist umso erstaunlicher, als Norpseudoephedrin in Tagesdosen appliziert werden muß, die durch transdermale Systeme in akzeptablen Größen normalerweise nicht erzielt werden können.

Dabei kann die wirkstoffundurchlässige Rückschicht aus flexiblem oder nicht flexiblem Material bestehen. Substanzen, die zu ihrer Herstellung verwendet werden können, sind Polymerfolien oder Metallfolien, wie Aluminiumfolie, die allein oder mit einem polymeren Substrat beschichtet, angewandt werden. Es können auch textile Flächengebilde verwendet werden, wenn die Bestandteile des Reservoir, aufgrund ihrer physikalischen Beschaffenheit durch sie nicht hindurchtreten können. Bei einer bevorzugten Ausführungsform ist die Rückschicht ein Verbundstoff, aus einer mit Aluminium bedampften Folie.

Die Reservoirschicht besteht aus einer Polymermatrix und dem Wirkstoff, wobei die Polymermatrix die Eigenschaft besitzt, den Zusammenhalt des Systems zu gewährleisten. Sie besteht aus einem Grundpolymer und gegebenenfalls den üblichen Zusätzen. Die Auswahl des Grundpolymers richtet sich nach den chemischen und physikalischen Eigenschaften des verwendeten Wirkstoffes. Beispielhafte Polymere sind Kautschuk, kautschukähnliche, synthetische Homo-, Co- oder Blockpolymere, Polyacrylsäureester und deren Copolymere, Polyurethane und Silikone. Grundsätzlich kommen alle Polymere in Frage, die bei der Herstellung von Haftklebern eingesetzt werden und physiologisch unbedenklich sind. Besonders bevorzugt sind solche, die aus Blockcopolymeren auf Basis von Styrol und 1,3-Dienen, Polyisobutylenen, Polymeren auf Acrylat- und/oder Methacrylat bestehen. Von den Blockcopolymeren auf Basis von Styrol und 1,3-Dienen werden ganz besonders lineare Styrol-Isopren-Blockcopolymeren eingesetzt.

Als Polymere auf Acrylat-Basis werden Acrylatcopolymere aus 2-Ethylhexylacrylat, Vinylacetat und Acrylsäure mit bzw. ohne Titanchelatester bevorzugt. Als Methacrylate werden Copolymere auf Basis von Dimethylaminoethylmethacrylate und neutralen Methacrylsäureestern bevorzugt. Als Ester von hydriertem Kolophonium werden vorzugsweise insbesondere dessen Methyl- und Glycerinester verwendet.

Die Art der möglichen Zusätze hängt vom eingesetzten Polymer und dem Wirkstoff ab: Nach ihrer Funktion lassen sich sich einteilen in Weichmacher, Klebrigmacher, Stabilisatoren, Trägerstoffe diffusions- und penetrationsregulierende Zusätze oder Füllstoffe. Die hierfür in Frage kommenden physiologisch unbedenklichen Substanzen sind dem Fachmann bekannt. Die Reservoirschicht besitzt eine solche Eigenklebrigkeit, daß ein dauernder Kontakt zur Haut sichergestellt ist.

Beispiele für geeignete Weichmacher sind Diester von Dicarbonsäuren, z.B. Di-n-butyladipat sowie Triglyceride, insbesondere mittelkettige Triglyceride der Capryl/Caprinsäure des Kokosöls zu nennen. Weitere Beispiele für einen geeigneten Weichmacher sind Glycerin, Propandiol (1,2) u.a..

Die ablösbare Schutzschicht, die mit der Reservoirschicht in Berührung steht und vor der Anwendung entfernt wird, besteht beispielsweise aus denselben Materialien, wie sie zur Herstellung der Rückschicht benutzt werden, vorausgesetzt, daß sie ablösbar gemacht werden, wie z.B. durch eine Siliconbehandlung. Andere ablösbare Schutzschichten sind z.B. Polytetrafluorethylen, behandeltes Papier, Cellophan, Polyvinylchlorid u.ä.. Wird das erfindungsgemäße Laminat vor Aufbringen der Schutzschicht in therapiegerechte Formate (Pflaster) aufgeteilt, so können die dann aufzubringenden Schutzschichtformate ein überstehendes Ende aufweisen, mit dessen Hilfe sie leichter von dem Pflaster abgezogen werden können.

Als Antiadipositum wird erfindungsgemäß Norpseudoephedrin eingesetzt, da es von allen Antiadiposita das geringste Abhängikeitspotential besitzt.

Das erfindungsgemäße transdermale therapeutische System wird hergestellt, indem der Wirkstoff zusammen mit den Bestandteilen der haftklebenden Reservoirschicht gegebenenfalls in Lösung homogen vermischt und auf die wirkstoffundurchlässige Rückschicht aufgestrichen wird, worauf gegebenenfalls das Lösemittel oder die Lösemittel entfernt wird/werden. Anschließend wird die Klebeschicht mit einer entsprechenden Schutzschicht versehen.

Die Erfindung wird durch die folgenden Beispiele erläutert:

### Rezepturbeispiel 1:

4,3 g n Heptan und 15,7 Butanon werden gemischt. Darin werden 4,0 g Norpseudoephedrin, freie Base, gelöst. Nach vollständiger Auflösung des Wirkstoffes gibt man portionsweise 23,5 g eines Glycerinesters von völlig hydriertem Kolophonium, 15,5 g eines linearen Styrol-Butadien-Styrol Blockcopolymers, 3,9 g Methylester von hydriertem Kolophonium und 3,1 g Triglyceride der Capryl/Caprinsäure des Kokosöls ("Mittelkettige Triglyceride" DAB 8), zu. Unter Lichtausschluß rührt man bis zur vollständigen Auflösung (ca. 8 Stunden) und streicht die erhaltene Lösung mit einem 300 µm Rakel auf eine aluminisierte und silikonisierte Polyethylen-Folie.

Nachdem das Lösemittel durch 25-minütiges Trocknen bei 50 °C im Trockenkanal entfernt wurde, deckt man den Klebfilm mit einer Polyester-Folie 15 µ ab. Mit geeigneten Schneidewerkzeugen stanzt man Flächen von 16 cm² bzw. 50 cm² aus und entfernt die Ränder durch Abgittern. Die in vitro-Freisetzungsgraphk ist in der Figur 1 wiedergegeben. Sie zeigt die kontrollierte Freisetzung des Wirkstoffes aus einem 16 cm² Pflaster in physiologischer Kochsalz-Lösung. Außerdem wurde von einem gesunden Freiwilligen ein 50 cm2 Pflaster 12 Stunden getragen, das bei einem Flächengewicht von 130 g/m² 52 mg Norpseudoephidrin, freie Base enthält. Nach dem Entfernen des Systems wurde sein Restgehalt mit ca. 17 mg bestimmt, so daß in einer Zeit von 12 Stunden ca. 35 mg/50 cm² durch menschliche Haut diffundiert sind.

Damit wird der therapeutisch geforderte Wert noch übertroffen.

### Rezepturbeispiel 2:

25 g Butanon und 15 g Ethylacetat werden gemischt. Darin werden 10,0 g Norpseudoephedrin, freie Base, gelöst. Nach vollständiger Auflösung des Wirkstoffs gibt man portionsweise 17,5 g eines Glycerinesters von völlig hydriertem Kolophonium und 22,5 g eines linearen Styrol-Butadien-Styrol Blockcopolymers hinzu. Unter Lichtausschluß rührt man bis zur vollständigen Auflösung (ca. acht Stunden) und streicht die erhaltene Lösung mit einem 350 µm Rakel auf eine aluminisierte und silikonisierte Polyethylen-Folie (Dicke: 100 µm).

Nachdem das Lösemittel durch 25-minütiges Trocknen bei 50°C im Trockenkanal entfernt wurde, deckt man den Klebefilm mit einer Polyethylenfolie (Dicke: 15µm) ab. Mit geeigneten Schneidewerkzeugen stanzt man Flächen von 16 cm² aus und entfernt die Ränder durch Abgittern. Die in vitro-Freisetzungskurve ist in Figur 2 wiedergegeben. Sie zeigt die kontrollierte Freisetzung des Wirkstoffs aus einem 16 cm² Pflaster in physiologischer Kochsalzlösung. Außerdem wurde die in vitro-Penetration an excidierter Mäusehaut bestimmt. Sie beträgt 9,8 mg/16 cm² x 24 h und liegt damit, ohne Weichmacherzusatz, in der Größenordnung von Rezepturbeispiel" 1.

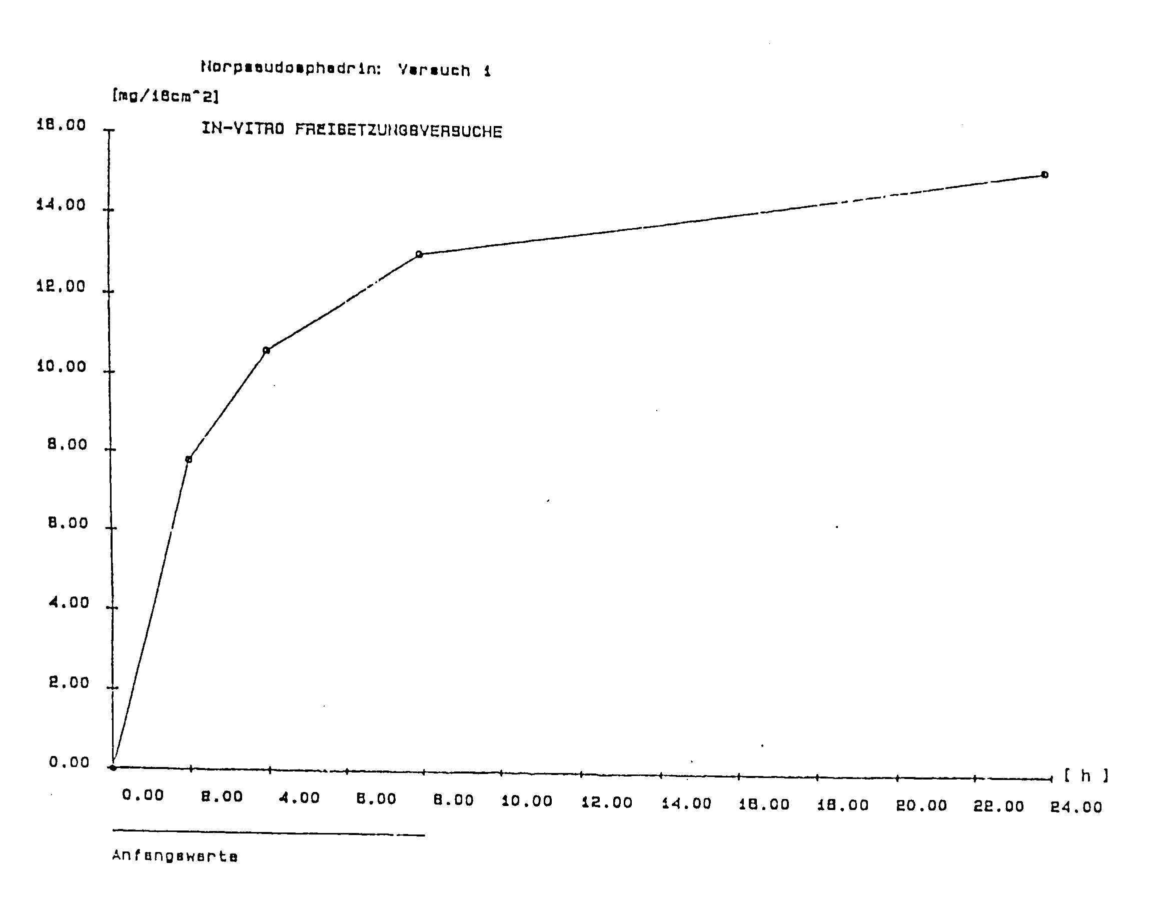

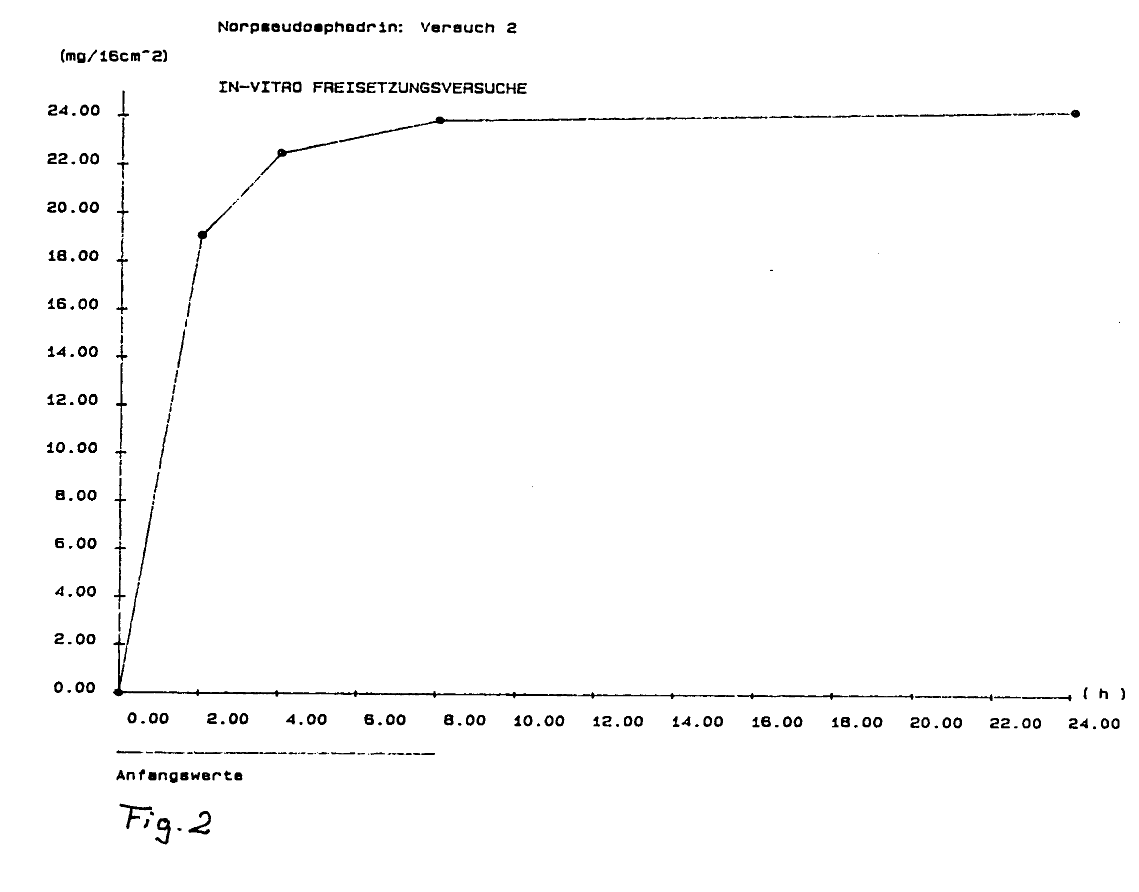

## Patentansprüche (Patentansprüche für folgende(n) Vertragsstaat(en): AT, BE, CH, DE, FR, GB, IT, LI, LU, NL, SE)

1. Transdermales therapeutisches System zur Verabreichung eines Antiadipositums an die Haut aus einer wirkstoffundurchlässigen Rückschicht, einer Reservoirschicht und gegebenenfalls einer wiederablösbaren Schutzschicht, dadurch gekennzeichnet, daß die Reservoirschicht haftklebend ist und 10 - 90 Gew.-% Polymermaterial, 0 - 30 Gew.-% Weichmacher und als Wirkstoff 0,1 - 20 Gew.-% Norpseudoephedrin enthält, wobei der Wirkstoff mit den Bestandteilen der haftklebenden Reservoirschicht in homogener Mischung vorliegt.

2. Transdermales therapeutisches System nach Anspruch 1, dadurch gekennzeichnet, daß das Polymermaterial ausgewählt ist aus der Gruppe, bestehend aus Blockcopolymeren auf Basis von Styrol und 1,3-Dienen, Polyisobutylenen, Polymeren auf Acrylat und/oder Methacrylatbasis und Estern von hydriertem Kolophonium.

3. Transdermales therapeutisches System nach Anspruch 2, dadurch gekennzeichnet, daß das Polymermaterial lineares Styrol-Isopren-Blockcopolymeres enthält.

4. Transdermales therapeutisches System nach Anspruch 2, dadurch gekennzeichnet, daß das Polymermaterial lineares Styrol-Butadien-Blockcopolymeres enthält.

5. Transdermales therapeutisches System nach Anspruch 2, dadurch gekennzeichnet, daß das Polymermaterial selbstvernetzendes Acrylatcopolymeres aus 2-Ethylhexylacrylat, Vinylacetat, Acrylsäure und Titanchelatester enthält.

6. Transdermales therapeutisches System nach Anspruch 2, dadurch gekennzeichnet, daß das Polymermaterial nicht selbstvernetzende Acrylatcopolymere aus 2-Ethylhexylacrylat, Vinylacetat und Acrylsäure enthält.

7. Transdermales therapeutisches System nach Anspruch 2, dadurch gekennzeichnet, daß das Polymermaterial als Polymer auf Basis von Methacrylaten ein Copolymer von Dimethylaminoethylmethacrylat und neutralen Methacrylsäureestern enthält.

8. Transdermales therapeutisches System nach Anspruch 2, dadurch gekennzeichnet, daß das Polymermaterial als Ester des hydrierten Kolophoniums dessen Methylester enthält.

9. Transdermales therapeutisches System nach Anspruch 2, dadurch gekennzeichnet, daß das Polymermaterial als Ester des hydrierten Kolophoniums dessen Glycerinester enthält.

10. Transdermales therapeutisches System nach Anspruch 1, dadurch gekennzeichnet, daß die Weichmacher ausgewählt sind aus der Gruppe, bestehend aus vicinalen Alkoholen und Estern.

11. Transdermales therapeutisches System nach Anspruch 10, dadurch gekennzeichnet, daß es als Weichmacher Propandiol-1,2 enthält.

12. Transdermales therapeutisches System nach Anspruch 10, dadurch gekennzeichnet, daß es als Weichmacher Di-n-butyladipat enthält.

13. Transdermales therapeutisches System nach Anspruch 10, dadurch gekennzeichnet, daß es als Weichmacher Triglyceride enthält.

14. Transdermales therapeutisches System nach Anspruch 10, dadurch gekennzeichnet, daß es als Weichmacher Glycerin enthält.

## Patentansprüche (Patentansprüche für folgende(n) Vertragsstaat(en): ES, GR)

1. Verfahren zur Herstellung eines transdermalen therapeutischen Systems zur Verabreichung eines Antiadipositums als Wirkstoff an die Haut, bestehend aus einer wirkstoffundurchlässigen Rückschicht, einer Reservoirschicht und gegebenenfalls einer wiederablösbaren Schutzschicht, dadurch gekennzeichnet, daß als Antiadipositum Norpseudoephedrin mit den Bestandteilen der Reservoirschicht, gegebenenfalls in Lösung, homogen vermischt, das Gemisch auf die wirkstoffundurchlässige Rückschicht aufgebracht, gegebenenfalls das Lösemittel entfernt und anschließend auf die Klebeschicht eine Schutzschicht aufgebracht wird, wobei die Reservoirschicht haftklebend ist und 10-90 Gew.-% Polymermaterial, 0-30 Gew.-% Weichmacher und 0,1-20 Gew.-% Norpseudoephedrin enthält.

2. Verfahren nach Anspruch 1, dadurch gekennzeichnet, daß das Polymermaterial ausgewählt ist aus der Gruppe, bestehend aus Blockcopolymeren auf Basis von Styrol und 1,3-Dienen, Polyisobutylenen, Polymeren auf Acrylat und/oder Methacrylatbasis und Estern von hydriertem Kolophonium.

3. Verfahren nach Anspruch 1, dadurch gekennzeichnet, daß das Polymermaterial lineares Styrol-Isopren-Blockcopolymeres enthält.

4. Verfahren nach Anspruch 1, dadurch gekennzeichnet, daß das Polymermaterial lineares Styrol-Butadien-Blockcopolymeres enthält.

5. Verfahren nach Anspruch 1, dadurch gekennzeichnet, daß das Polymermaterial selbstvernetzendes Acrylatcopolymeres aus 2-Ethylhexylacrylat, Vinylacetat, Acrylsäure und Titanchelatester enthält.

6. Verfahren nach Anspruch 1, dadurch gekennzeichnet, daß das Polymermaterial nicht selbstvernetzende Acrylatcopolymere aus 2-Ethylhexylacrylat, Vinylacetat und Acrylsäure enthält.

7. Verfahren nach Anspruch 1, dadurch gekennzeichnet, daß das Polymermaterial als Polymer auf Basis von Methacrylaten ein Copolymer von Dimethylaminoethylmethacrylat und neutralen Methacrylsäureestern enthält.

8. Verfahren nach Anspruch 1, dadurch gekennzeichnet, daß das Polymermaterial als Ester des hydrierten Kolophoniums dessen Methylester enthält.

9. Verfahren nach Anspruch 1, dadurch gekennzeichnet, daß das Polymermaterial als Ester des hydrierten Kolophoniums dessen Glycerinester enthält.

10. Verfahren nach Anspruch 1, dadurch gekennzeichnet, daß die Weichmacher ausgewählt sind aus der Gruppe, bestehend aus vicinalen Alkoholen und Estern.

11. Verfahren nach Anspruch 10, dadurch gekennzeichnet, daß es als Weichmacher Propandiol-1,2 enthält.

12. Verfahren nach Anspruch 10, dadurch gekennzeichnet, daß es als Weichmacher Di-n-butyladipat enthält.

13. Verfahren nach Anspruch 10, dadurch gekennzeichnet, daß es als Weichmacher Triglyceride enthält.

14. Verfahren nach Anspruch 10, dadurch gekennzeichnet, daß es als Weichmacher Glycerin enthält.

## Claims (Claims for the following Contracting State(s): AT, BE, CH, DE, FR, GB, IT, LI, NL, SE)

1. A transdermal therapeutical system for the administration of an antiadipogenic to the skin comprising a backing layer being impermeable to the active substance, a reservoir layer, and optionally a removable protective layer, characterized in that the reservoir layer is pressure-sensitive adhesive and comprises 10-90%-wt polymeric material, 0-30%-wt softener and, as active substance, 0.1-20%-wt norpseudoephedrine, whereby the active substance is present in homogenous mixture with the components of the pressure-sensitive adhesive reservoir layer.

2. The transdermal therapeutical system according to claim 1, characterized in that the polymeric material is selected from the group consisting of block copolymers on the basis of styrene and 1,3-dienes, polyisobutylenes, polymers on the basis of acrylate and/or methacrylate, and esters of hydrogenated colophonium.

3. The transdermal therapeutical system according to claim 2, characterized in that the polymeric material comprises linear styrene-isoprene block copolymer.

4. The transdermal therapeutical system according to claim 2, characterized in that the polymeric material comprises linear styrene-butadiene block copolymer.

5. The transdermal therapeutical system according to claim 2, characterized in that the polymeric material comprises self-crosslinking acrylate copolymer of 2-ethylhexyl acrylate, vinyl acetate, acrylic acid, and titanium chelate ester.

6. The transdermal therapeutical system according to claim 2, characterized in that the polymeric material comprises non-self-crosslinking acrylate copolymers of 2-ethylhexyl acrylate, vinyl acetate, and acrylic acid.

7. The transdermal therapeutical system according to claim 2, characterized in that the polymeric material comprises as polymer on the basis of methacrylates a copolymer of dimethylaminoethyl methacrylate and neutral methacrylic acid esters.

8. The transdermal therapeutical system according to claim 2, characterized in that the polymeric material comprises as ester of the hydrogenated colophonium the methylesters thereof.

9. The transdermal therapeutical system according to claim 2, characterized in that the polymeric material comprises as ester of the hydrogenated colophonium the glyceryl esters thereof.

10. The transdermal therapeutical system according to claim 1, characterized in that the softeners are selected from the group consisting of vicinal alcohols and esters.

11. The transdermal therapeutical system according to claim 10, characterized in that it comprises propanediol-1,2 as softener.

12. The transdermal therapeutical system according to claim 10, characterized in that it comprises di-n-butyl adipate as softener.

13. The transdermal therapeutical system according to claim 10, characterized in that it comprises triglycerides as softener.

14. The transdermal therapeutical system according to claim 10, characterized in that it comprises glycerol as softener.

## Claims (Claims for the following Contracting State(s): ES, GR)

1. A process for the production of a transdermal therapeutic system for the administration of an antiadipogenic to the skin comprising a backing layer being impermeable to the active substance, a reservoir layer, and optionally a removable protective layer, characterized in that as antiadipogenic norpseudoephedrine is mixed homogeneously with the components of the reservoir layer, optionally in solution; the mixture is applied onto the active substance-impermeable backing layer; the solvent is removed if required, and subsequently a protective layer is applied onto the adhesive layer, whereby the reservoir layer is pressure-sensitive adhesive and comprises 10-90%-wt of polymeric material, 0-30%-wt of softeners and 0.1-20%-wt norpseudoephedrine.

2. The process according to claim 1, characterized in that the polymeric material is selected from the group consisting of block copolymers on the basis of styrene and 1,3-dienes, polyisobutylenes, polymers on the basis of acrylate and/or methacrylate, and esters of hydrogenated colophonium.

3. The process according to claim 1, characterized in that the polymeric material comprises linear styrene-isoprene block copolymer.

4. The process according to claim 1, characterized in that the polymeric material comprises linear styrene-butadiene block copolymer.

5. The process according to claim 1, characterized in that the polymeric material comprises self-crosslinking acrylate copolymer of 2-ethylhexyl acrylate, vinyl acetate, acrylic acid, and titanium chelate ester.

6. The process according to claim 1, characterized in that the polymeric material comprises non-self-crosslinking acrylate copolymers of 2-ethylhexyl acrylate, vinyl acetate, and acrylic acid.

7. The process according to claim 1, characterized in that the polymeric material comprises as polymer on the basis of methacrylates a copolymer of dimethylaminoethyl methacrylate and neutral methacrylic acid esters.

8. The process according to claim 1, characterized in that the polymeric material comprises as ester of the hydrogenated colophonium the methylesters thereof.

9. The process according to claim 1, characterized in that the polymeric material comprises as ester of the hydrogenated colophonium the glyceryl esters thereof.

10. The process according to claim 1, characterized in that the softeners are selected from the group consisting of vicinal alcohols and esters.

11. The process according to claim 10, characterized in that it comprises propanediol-1,2 as softener.

12. The process according to claim 10, characterized in that it comprises di-n-butyl adipate as softener.

13. The process according to claim 10, characterized in that it comprises triglycerides as softener.

14. The process according to claim 10, characterized in that it comprises glycerol as softener.

## Revendications (Revendications pour l'(les) Etat(s) contractant(s) suivant(s): AT, BE, CH, DE, FR, GB, IT, LI, NL, SE)

1. Système thérapeutique transdermique pour l'administration d'une substance antiadiposité par la peau, constitué d'une couche dorsale imperméable au principe actif, d'une couche formant réservoir et, éventuellement d'une couche protectrice amovible, caractérisé en ce que la couche formant réservoir est autocollante et contient 10 à 90% en poids de matière polymérique, 0 à 30% en poids de plastifiant et, à titre de principe actif, 0,1 à 20% en poids de norpseudoéphédrine, où le principe actif se présente en mélange homogène avec les constituants de la couche formant réservoir autocollante.

2. Système thérapeutique transdermique suivant la revendication 1, caractérisé en ce que l'on choisit la matière polymérique dans le groupe formé par des copolymères séquences à base de styrène et de 1,3-diènes, de polyisobutylènes, de polymères à base d'acrylate et/ou de méthacrylate et d'esters de la colophane hydrogénée.

3. Système thérapeutique transdermique suivant la revendication 2, caractérisé en ce que la matière polymérique contient un copolymère séquencé de styrène et d'isoprène, linéaire.

4. Système thérapeutique transdermique suivant la revendication 2, caractérisé en ce que la matière polymérique contient un copolymère séquencé de styrène et de butadiène, linéaire.

5. Système thérapeutique transdermique suivant la revendication 2, caractérisé en ce que la matière polymérique contient un copolymère d'acrylate autoréticulant constitué d'acrylate de 2-éthylhexyle, d'acétate de vinyle, d'acide acrylique et d'un chélate de titane.

6. Système thérapeutique transdermique suivant la revendication 2, caractérisé en ce que la matière polymérique contient des copolymères d'acrylate non autoréticulants constitués d'acrylate de 2-éthylhexyle, d'acétate de vinyle et d'acide acrylique.

7. Système thérapeutique transdermique suivant la revendication 2, caractérisé en ce que la matière polymérique contient, à titre de polymère à base de méthacrylates, un copolymère de méthacrylate de diméthylaminoéthyle et d'esters de l'acide méthacrylique neutres.

8. Système thérapeutique transdermique suivant la revendication 2, caractérisé en ce que la matière polymérique contient, à titre d'ester de la colophane hydrogénée, son ester méthylique.

9. Système thérapeutique transdermique suivant la revendication 2, caractérisé en ce que la matière polymérique contient, à titre d'ester de la colophane hydrogénée, son ester de la glycérine.

10. Système thérapeutique transdermique suivant la revendication 1, caractérisé en ce que l'on choisit les plastifiants dans le groupe formé par les esters et alcools vicinaux.

11. Système thérapeutique transdermique suivant la revendication 10, caractérisé en ce qu'il contient le propanediol-1,2 à titre de plastifiant.

12. Système thérapeutique transdermique suivant la revendication 10, caractérisé en ce qu'il contient l' adipate de di-n-butyle à titre de plastifiant.

13. Système thérapeutique transdermique suivant la revendication 10, caractérisé en ce qu'il contient des triglycérides à titre de plastifiants.

14. Système thérapeutique transdermique suivant la revendication 10, caractérisé en ce qu'il contient de la glycérine à titre de plastifiant.

## Revendications (Revendications pour l'(les) Etat(s) contractant(s) suivant(s): ES, GR)

1. Procédé de préparation d'un système thérapeutique transdermique pour l'administration d'une substance antiadiposité par la peau, constitué d'une couche dorsale imperméable au principe actif, d'une couche formant réservoir et, éventuellement d'une couche protectrice amovible, caractérisé en ce que l'on mélange de manière homogène, éventuellement en solution, la norpseudoéphédrine servant de substance antiadiposité aux constituants de la couche formant réservoir, on applique le mélange sur la couche dorsale imperméable au principe actif, on élimine éventuellement le solvant et on applique finalement une couche protectrice sur la couche collante, où la couche formant réservoir est autocollante et contient 10 à 90 % en poids de matière polymérique, 0 à 30 % en poids de plastifiant et 0,1 à 20 % en poids de norpseudoéphédrine.

2. Procédé de préparation d'un système thérapeutique transdermique suivant la revendication 1, caractérisé en ce que l'on choisit la matière polymérique dans le groupe formé par des copolymères séquencés à base de styrène et de 1,3-diènes, de polyisobutylènes, de polymères à base d'acrylate et/ou de méthacrylate et d'esters de la colophane hydrogénée.

3. Procédé de préparation d'un système thérapeutique transdermique suivant la revendication 2, caractérisé en ce que la matière polymérique contient un copolymère séquencé de styrène et d'isoprène, linéaire.

4. Procédé de préparation d'un système thérapeutique transdermique suivant la revendication 2, caractérisé en ce que la matière polymérique contient un copolymère séquencé de styrène et de butadiène, linéaire.

5. Procédé de préparation d'un système thérapeutique transdermique suivant la revendication 2, caractérisé en ce que la matière polymérique contient un copolymère d'acrylate autoréticulant constitué d'acrylate de 2-éthylhexyle, d'acétate de vinyle, d'acide acrylique et d'un chélate de titane.

6. Procédé de préparation d'un système thérapeutique transdermique suivant la revendication 2, caractérisé en ce que la matière polymérique contient des copolymères d'acrylate non autoréticulants constitués d'acrylate des 2-éthylhexyle, d'acétate de vinyle et d'acide acrylique.

7. Procédé de préparation d'un système thérapeutique transdermique suivant la revendication 2, caracterisé en ce que la matière polymérique contient, à titre de polymère à base de méthacrylates, un copolymère de méthacrylate de diméthylaminoéthyle et d'esters de l'acide méthacrylique neutres.

8. Procédé de préparation d'un système thérapeutique transdermique suivant la revendication 2, caractérisé en ce que la matière polymérique contient, à titre d'ester de la colophane hydrogénée, son ester méthylique.

9. Procédé de préparation d'un système thérapeutique transdermique suivant la revendication 2, caractérisé en ce que la matière polymérique contient, à titre d'ester de la colophane hydrogénée, son ester de la glycérine.

10. Procédé de préparation d'un système thérapeutique transdermique suivant la revendication 1, caractérisé en ce que l'on choisit les plastifiants dans le groupe formé par les esters et alcools vicinaux.

11. Procédé de préparation d'un système thérapeutique transdermique suivant la revendication 10, caractérisé en ce qu'il contient le propanediol-1,2 à titre de plastifiant.

12. Procédé de préparation d'un système thérapeutique transdermique suivant la revendication 10, caractérisé en ce qu'il contient l'adipate de di-n-butyle à titre de plastifiant.

13. Procédé de préparation d'un système thérapeutique transdermique suivant la revendication 10, caractérisé en ce ou'il contient des triglycérides à titre de plastifiants

14. Procédé de préparation d'un système thérapeutique transdermique suivant la revendication 10, caractérisé en ce qu'il contient de la glycérine à titre de plastifiant.
